(19) Europäisches Patentamt
European Patent Office
Office européen des brevets

(11) **EP 1 345 969 B1**

(12) **FASCICULE DE BREVET EUROPEEN**

(45) Date de publication et mention
de la délivrance du brevet:
**11.08.2010 Bulletin 2010/32**

(21) Numéro de dépôt: **01995797.6**

(22) Date de dépôt: **26.12.2001**

(51) Int Cl.:
*C07K 16/28* (2006.01)  *C12N 15/13* (2006.01)
*C12N 15/63* (2006.01)  *C12N 5/20* (2006.01)
*A61K 39/395* (2006.01)  *A61P 37/06* (2006.01)
*A61P 37/08* (2006.01)  *A61P 29/00* (2006.01)

(86) Numéro de dépôt international:
**PCT/FR2001/004203**

(87) Numéro de publication internationale:
**WO 2002/051871 (04.07.2002 Gazette 2002/27)**

(54) **ANTICORPS ANTI-CD28**

ANTIKÖRPER GEGEN CD28

ANTI-CD28 ANTIBODY

(84) Etats contractants désignés:
**AT BE CH CY DE DK ES FI FR GB GR IE IT LI LU MC NL PT SE TR**

(30) Priorité: **26.12.2000 FR 0017025**

(43) Date de publication de la demande:
**24.09.2003 Bulletin 2003/39**

(73) Titulaire: **INSTITUT NATIONAL DE LA SANTE ET DE LA RECHERCHE MEDICALE (INSERM)**
**75013 Paris (FR)**

(72) Inventeurs:
• **SOULILLOU, Jean-Paul**
**44100 Nantes (FR)**
• **LAFLAMME, Geneviève**
**G8Y 3P8 Quebec (CA)**
• **VANHOVE, Bernard**
**44400 REZE (FR)**
• **OLIVE, Daniel**
**13009 Marseille (FR)**

(74) Mandataire: **Vialle-Presles, Marie José et al Cabinet ORES**
**36, rue de St Pétersbourg**
**75008 Paris (FR)**

(56) Documents cités:
**EP-A- 0 440 373    WO-A-94/28912**

• **TAN P. ET AL.: "Humanization of an anti-CD28 antibody using germline human antibody sequences." BLOOD (2000 NOV) 96 (11 PART 1) 31A., XP002177441**
• **NUNES J ET AL: "CD28 mAbs with distinct binding properties differ in their ability to induce T cell activation: analysis of early and late activation events." INTERNATIONAL IMMUNOLOGY, (1993 MAR) 5 (3) 311-5., XP001024214 cité dans la demande**
• **GILLILAND L. K. ET AL.: "Rapid and reliable cloning of antibody variable regions and generation of recombinant single chain antibody fragments." TISSUE ANTIGENS, (1996) 47 (1) 1-20., XP000574883**
• **HASPOT FABIENNE ET AL: "Differential effect of CD28 versus B7 blockade on direct pathway of allorecognition and self-restricted responses." BLOOD. UNITED STATES 15 MAR 2002, vol. 99, no. 6, 15 mars 2002 (2002-03-15), pages 2228-2234, XP002201786 ISSN: 0006-4971**
• **RAAG; WHITLOW: 'Single-chain Fvs' FASEB J. vol. 9, 1995, pages 73 - 80**

LISTE DE SEQUENCES

<110> INSERM
IMTIXT SANGSTAT
LAFLAMME, Geneviève
VANHOVE, Bernard

<120> ANTICORPS ANTI-CD28

<130> MJPcb598-48

<140>
<141>

<150> 00 17025
<151> 2000-12-26

<160> 4

<170> PatentIn Ver. 2.1

<210> 1
<211> 732
<212> ADN
<213> Séquence artificielle

<220>
<223> Description de la séquence artificielle: fragment scFv

<220>
<221> CDS
<222> (1)..(732)

<220>
<221> misc_feature
<222> (1)..(360)
<223> chaîne légère

<220>

<221> misc_feature
<222> (406)..(732)
<223> chaîne lourde

<220>
<221> misc_feature
<222> (361)..(405)
<223> lieur

<220>
<221> misc_feature
<222> (85)..(96)
<223> CDR1

<220>
<221> misc_feature
<222> (139)..(189)
<223> CDR2

<220>
<221> misc_feature
<222> (286)..(324)
<223> CDR3

<220>
<221> misc_feature
<222> (496)..(522)
<223> CDR1

<220>
<221> misc_feature
<222> (553)..(576)
<223> CDR2

<220>
<221> misc_feature
<222> (673)..(681)
<223> CDR3

<400> 1

```
gtc aag ctg cag cag tca gga gct gag ctg gtg aaa ccc ggg gcg tcg      48
Val Lys Leu Gln Gln Ser Gly Ala Glu Leu Val Lys Pro Gly Ala Ser
 1               5                   10                  15

gtg agg ctg tcc tgc aag gcg tct ggt tac acc ttc act gaa tat att      96
Val Arg Leu Ser Cys Lys Ala Ser Gly Tyr Thr Phe Thr Glu Tyr Ile
            20                  25                  30

ata cac tgg ata aag ctg agg tct gga cag ggt ctt gag tgg att ggg     144
Ile His Trp Ile Lys Leu Arg Ser Gly Gln Gly Leu Glu Trp Ile Gly
        35                  40                  45

tgg ttt tac cct gga agt aat gat ata cag tac aat gcg aaa ttc aag     192
Trp Phe Tyr Pro Gly Ser Asn Asp Ile Gln Tyr Asn Ala Lys Phe Lys
    50                  55                  60

ggc aag gcc aca ttg act gcg gac aaa tcc tcc agc acc gtc tat atg     240
Gly Lys Ala Thr Leu Thr Ala Asp Lys Ser Ser Ser Thr Val Tyr Met
65                  70                  75                  80

gaa ctt act gga ttg aca tct gag gac tct gcg gtc tat ttc tgt gca     288
Glu Leu Thr Gly Leu Thr Ser Glu Asp Ser Ala Val Tyr Phe Cys Ala
                85                  90                  95

aga cgc gac gat ttc tct ggt tac gac gcc ctt cct tac tgg ggc caa     336
Arg Arg Asp Asp Phe Ser Gly Tyr Asp Ala Leu Pro Tyr Trp Gly Gln
            100                 105                 110

ggg acc atg gtc acc gtc tcc tca ggt gga ggc ggt tca ggc gga ggt     384
Gly Thr Met Val Thr Val Ser Ser Gly Gly Gly Gly Ser Gly Gly Gly
            115                 120                 125

ggc tct ggc ggt ggc gga tcg gac atc cag atg acc cag tct cca gcc     432
Gly Ser Gly Gly Gly Gly Ser Asp Ile Gln Met Thr Gln Ser Pro Ala
        130                 135                 140

tcc cta tct gtt tct gtg gga gaa act gtc acc atc acg tgt cga aca     480
Ser Leu Ser Val Ser Val Gly Glu Thr Val Thr Ile Thr Cys Arg Thr
145                 150                 155                 160
```

```
aat gaa aat att tac agt aat tta gca tgg tat cag cag aaa cag gga    528
Asn Glu Asn Ile Tyr Ser Asn Leu Ala Trp Tyr Gln Gln Lys Gln Gly
                165             170                 175


aaa tct cct cag ctc ctg atc tat gct gca aca cac tta gta gag ggt    576
Lys Ser Pro Gln Leu Leu Ile Tyr Ala Ala Thr His Leu Val Glu Gly
            180             185                 190

gtg cca tca agg ttc agt ggc agt gga tca ggc aca cag tat tcc ctc    624
Val Pro Ser Arg Phe Ser Gly Ser Gly Ser Gly Thr Gln Tyr Ser Leu
            195             200                 205

aag atc acc agc ctg cag tct gaa gat ttt ggg aat tat tac tgt caa    672
Lys Ile Thr Ser Leu Gln Ser Glu Asp Phe Gly Asn Tyr Tyr Cys Gln
            210             215                 220

cac ttt tgg ggt act ccg tgc acg ttc gga ggg ggg acc aag ctg gaa    720
His Phe Trp Gly Thr Pro Cys Thr Phe Gly Gly Gly Thr Lys Leu Glu
225             230             235                 240

ata aaa cgg act                                                    732
Ile Lys Arg Thr
```

<210> 2
<211> 244
<212> PRT
<213> Séquence artificielle
<223> Description de la séquence artificielle: fragment scFv

<400> 2

```
Val Lys Leu Gln Gln Ser Gly Ala Glu Leu Val Lys Pro Gly Ala Ser
 1               5                  10                  15

Val Arg Leu Ser Cys Lys Ala Ser Gly Tyr Thr Phe Thr Glu Tyr Ile
            20                  25                  30

Ile His Trp Ile Lys Leu Arg Ser Gly Gln Gly Leu Glu Trp Ile Gly
        35                  40                  45

Trp Phe Tyr Pro Gly Ser Asn Asp Ile Gln Tyr Asn Ala Lys Phe Lys
    50                  55                  60

Gly Lys Ala Thr Leu Thr Ala Asp Lys Ser Ser Ser Thr Val Tyr Met
 65                  70                  75                  80

Glu Leu Thr Gly Leu Thr Ser Glu Asp Ser Ala Val Tyr Phe Cys Ala
                85                  90                  95

Arg Arg Asp Asp Phe Ser Gly Tyr Asp Ala Leu Pro Tyr Trp Gly Gln
            100                 105                 110

Gly Thr Met Val Thr Val Ser Ser Gly Gly Gly Gly Ser Gly Gly Gly
        115                 120                 125

Gly Ser Gly Gly Gly Gly Ser Asp Ile Gln Met Thr Gln Ser Pro Ala
    130                 135                 140
```

```
Ser Leu Ser Val Ser Val Gly Glu Thr Val Thr Ile Thr Cys Arg Thr
145                 150                 155                 160

Asn Glu Asn Ile Tyr Ser Asn Leu Ala Trp Tyr Gln Gln Lys Gln Gly
                165                 170                 175

Lys Ser Pro Gln Leu Leu Ile Tyr Ala Ala Thr His Leu Val Glu Gly
            180                 185                 190

Val Pro Ser Arg Phe Ser Gly Ser Gly Ser Gly Thr Gln Tyr Ser Leu
            195                 200                 205

Lys Ile Thr Ser Leu Gln Ser Glu Asp Phe Gly Asn Tyr Tyr Cys Gln
        210                 215                 220

His Phe Trp Gly Thr Pro Cys Thr Phe Gly Gly Gly Thr Lys Leu Glu
225                 230                 235                 240

Ile Lys Arg Thr
```

<210> 3
<211> 2013
<212> ADN
<213> Séquence artificielle

<220>
<223> Description de la séquence artificielle: fusion scFv anti-CD28/alpha-1 antitrypsine

<220>
<221> CDS
<222> (1)..(2013)

<220>
<221> misc_signal
<222> (1)..(57)
<223> signal Omp A

<220>
<221> misc_feature
<222> (64)..(75)
<223> Flag Tag

<220>
<221> misc_feature
<222> (109)..(810)
<223> scFv 28.3

<220>
<221> misc_feature
<222> (826)..(1992)
<223> alpha-1 antitrypsine

<220>
<221> misc_feature
<222> (1993)..(2007)
<223> His Tag

<400> 3

```
atg aaa aag aca gct atc gcg att gca gtg gca ctg gct ggt ttc gct      48
Met Lys Lys Thr Ala Ile Ala Ile Ala Val Ala Leu Ala Gly Phe Ala
 1               5                   10                  15

acc gta gcg cag gcc gac tac aaa gat atc gtc aag ctg cag cag tca      96
Thr Val Ala Gln Ala Asp Tyr Lys Asp Ile Val Lys Leu Gln Gln Ser
                20                  25                  30

gga gct gag ctg gtg aaa ccc ggg gcg tcg gtg agg ctg tcc tgc aag     144
Gly Ala Glu Leu Val Lys Pro Gly Ala Ser Val Arg Leu Ser Cys Lys
            35                  40                  45

gcg tct ggt tac acc ttc act gaa tat att ata cac tgg ata aag ctg     192
Ala Ser Gly Tyr Thr Phe Thr Glu Tyr Ile Ile His Trp Ile Lys Leu
        50                  55                  60

agg tct gga cag ggt ctt gag tgg att ggg tgg ttt tac cct gga agt     240
Arg Ser Gly Gln Gly Leu Glu Trp Ile Gly Trp Phe Tyr Pro Gly Ser
 65                  70                  75                  80

aat gat ata cag tac aat gcg aaa ttc aag ggc aag gcc aca ttg act     288
Asn Asp Ile Gln Tyr Asn Ala Lys Phe Lys Gly Lys Ala Thr Leu Thr
                85                  90                  95

gcg gac aaa tcc tcc agc acc gtc tat atg gaa ctt act gga ttg aca     336
Ala Asp Lys Ser Ser Ser Thr Val Tyr Met Glu Leu Thr Gly Leu Thr
                100                 105                 110

tct gag gac tct gcg gtc tat ttc tgt gca aga cgc gac gat ttc tct     384
Ser Glu Asp Ser Ala Val Tyr Phe Cys Ala Arg Arg Asp Asp Phe Ser
            115                 120                 125

ggt tac gac gcc ctt cct tac tgg ggc caa ggg acc atg gtc acc gtc     432
Gly Tyr Asp Ala Leu Pro Tyr Trp Gly Gln Gly Thr Met Val Thr Val
        130                 135                 140

tcc tca ggt gga ggc ggt tca ggc gga ggt ggc tct ggc ggt ggc gga     480
Ser Ser Gly Gly Gly Gly Ser Gly Gly Gly Gly Ser Gly Gly Gly Gly
145                 150                 155                 160

tcg gac atc cag atg acc cag tct cca gcc tcc cta tct gtt tct gtg     528
Ser Asp Ile Gln Met Thr Gln Ser Pro Ala Ser Leu Ser Val Ser Val
                165                 170                 175

gga gaa act gtc acc atc acg tgt cga aca aat gaa aat att tac agt     576
Gly Glu Thr Val Thr Ile Thr Cys Arg Thr Asn Glu Asn Ile Tyr Ser
                180                 185                 190

aat tta gca tgg tat cag cag aaa cag gga aaa tct cct cag ctc ctg     624
Asn Leu Ala Trp Tyr Gln Gln Lys Gln Gly Lys Ser Pro Gln Leu Leu
            195                 200                 205

atc tat gct gca aca cac tta gta gag ggt gtg cca tca agg ttc agt     672
Ile Tyr Ala Ala Thr His Leu Val Glu Gly Val Pro Ser Arg Phe Ser
        210                 215                 220

ggc agt gga tca ggc aca cag tat tcc ctc aag atc acc agc ctg cag     720
Gly Ser Gly Ser Gly Thr Gln Tyr Ser Leu Lys Ile Thr Ser Leu Gln
225                 230                 235                 240
```

```
tct gaa gat ttt ggg aat tat tac tgt caa cac ttt tgg ggt act ccg   768
Ser Glu Asp Phe Gly Asn Tyr Tyr Cys Gln His Phe Trp Gly Thr Pro
            245             250                 255

tgc acg ttc gga ggg ggg acc aag ctg gaa ata aaa cgg act gtg gct   816
Cys Thr Phe Gly Gly Gly Thr Lys Leu Glu Ile Lys Arg Thr Val Ala
            260             265                 270

gca cca tct gaa ttc aac aag atc acc ccc aac ctg gct gag ttc gcc   864
Ala Pro Ser Glu Phe Asn Lys Ile Thr Pro Asn Leu Ala Glu Phe Ala
            275             280                 285

ttc agc cta tac cgc cag ctg gca cac cag tcc aac agc acc aat atc   912
Phe Ser Leu Tyr Arg Gln Leu Ala His Gln Ser Asn Ser Thr Asn Ile
            290             295                 300

ttc ttc tcc cca gtg agc atc gct aca gcc ttt gca atg ctc tcc ctg   960
Phe Phe Ser Pro Val Ser Ile Ala Thr Ala Phe Ala Met Leu Ser Leu
305             310             315                 320

ggg acc aag gct gac act cac gat gaa atc ctg gag ggc ctg aat ttc  1008
Gly Thr Lys Ala Asp Thr His Asp Glu Ile Leu Glu Gly Leu Asn Phe
            325             330                 335

aac ctc acg gag att ccg gag gct cag atc cat gaa ggc ttc cag gaa  1056
Asn Leu Thr Glu Ile Pro Glu Ala Gln Ile His Glu Gly Phe Gln Glu
            340             345                 350

ctc ctc cgt acc ctc aac cag cca gac agc cag ctc cag ctg acc acc  1104
Leu Leu Arg Thr Leu Asn Gln Pro Asp Ser Gln Leu Gln Leu Thr Thr
            355             360                 365

ggc aat ggc ctg ttc ctc agc gag ggc ctg aag cta gtg gat aag ttt  1152
Gly Asn Gly Leu Phe Leu Ser Glu Gly Leu Lys Leu Val Asp Lys Phe
            370             375                 380

ttg gag gat gtt aaa aag ttg tac cac tca gaa gcc ttc act gtc aac  1200
Leu Glu Asp Val Lys Lys Leu Tyr His Ser Glu Ala Phe Thr Val Asn
385             390             395                 400

ttc ggg gac acc gaa gag gcc aag aaa cag atc aac gat tac gtg gag  1248
Phe Gly Asp Thr Glu Glu Ala Lys Lys Gln Ile Asn Asp Tyr Val Glu
            405             410                 415

aag ggt act caa ggg aaa att gtg gat ttg gtc aag gag ctt gac aga  1296
Lys Gly Thr Gln Gly Lys Ile Val Asp Leu Val Lys Glu Leu Asp Arg
            420             425                 430

gac aca gtt ttt gct ctg gtg aat tac atc ttc ttt aaa ggc aaa tgg  1344
Asp Thr Val Phe Ala Leu Val Asn Tyr Ile Phe Phe Lys Gly Lys Trp
            435             440                 445

gag aga ccc ttt gaa gtc aag gac acc gag gaa gag gac ttc cac gtg  1392
Glu Arg Pro Phe Glu Val Lys Asp Thr Glu Glu Glu Asp Phe His Val
            450             455                 460

gac cag gtg acc acc gtg aag gtg cct atg atg aag cgt tta ggc atg  1440
Asp Gln Val Thr Thr Val Lys Val Pro Met Met Lys Arg Leu Gly Met
465             470             475                 480
```

17

```
ttt aac atc cag cac tgt aag aag ctg tcc agc tgg gtg ctg ctg atg    1488
Phe Asn Ile Gln His Cys Lys Lys Leu Ser Ser Trp Val Leu Leu Met
                485             490                 495

aaa tac ctg ggc aat gcc acc gcc atc ttc ttc ctg cct gat gag ggg    1536
Lys Tyr Leu Gly Asn Ala Thr Ala Ile Phe Phe Leu Pro Asp Glu Gly
                500             505                 510

aaa cta cag cac ctg gaa aat gaa ctc acc cac gat atc atc acc aag    1584
Lys Leu Gln His Leu Glu Asn Glu Leu Thr His Asp Ile Ile Thr Lys
                515             520                 525

ttc ctg gaa aat gaa gac aga agg tct gcc agc tta cat tta ccc aaa    1632
Phe Leu Glu Asn Glu Asp Arg Arg Ser Ala Ser Leu His Leu Pro Lys
        530             535                 540

ctg tcc att act gga acc tat gat ctg aag agc gtc ctg ggt caa ctg    1680
Leu Ser Ile Thr Gly Thr Tyr Asp Leu Lys Ser Val Leu Gly Gln Leu
545             550                 555                 560

ggc atc act aag gtc ttc agc aat ggg gct gac ctc tcc ggg gtc aca    1728
Gly Ile Thr Lys Val Phe Ser Asn Gly Ala Asp Leu Ser Gly Val Thr
                565             570                 575

gag gag gca ccc ctg aag ctc tcc aag gcc gtg cat aag gct gtg ctg    1776
Glu Glu Ala Pro Leu Lys Leu Ser Lys Ala Val His Lys Ala Val Leu
                580             585                 590

acc atc gac gag aaa ggg act gaa gct gct ggg gcc atg ttt tta gag    1824
Thr Ile Asp Glu Lys Gly Thr Glu Ala Ala Gly Ala Met Phe Leu Glu
                595             600                 605

gcc ata ccc atg tct atc ccc ccc gag gtc aag ttc aac aaa ccc ttt    1872
Ala Ile Pro Met Ser Ile Pro Pro Glu Val Lys Phe Asn Lys Pro Phe
        610             615                 620

gtc ttc tta atg att gaa caa aat acc aag tct ccc ctc ttc atg gga    1920
Val Phe Leu Met Ile Glu Gln Asn Thr Lys Ser Pro Leu Phe Met Gly
625             630                 635                 640

aaa gtg gtg aat ccc acc caa aaa ctc gag gga gaa ttc gaa cag aaa    1968
Lys Val Val Asn Pro Thr Gln Lys Leu Glu Gly Glu Phe Glu Gln Lys
                645             650                 655

ctg atc tct gaa gaa gac ctg aac cac cac cac cac cac tga taa    2013
Leu Ile Ser Glu Glu Asp Leu Asn His His His His His
        660             665                 670
```

<210> 4
<211> 669
<212> PRT
<213> Séquence artificielle

<223> Description de la séquence artificielle: fusion scFv anti-CD28/alpha-1 antitrypsine

<400> 4

```
Met Lys Lys Thr Ala Ile Ala Ile Ala Val Ala Leu Ala Gly Phe Ala
     1                5                   10                    15
```

```
Thr Val Ala Gln Ala Asp Tyr Lys Asp Ile Val Lys Leu Gln Gln Ser
        20                  25                  30
Gly Ala Glu Leu Val Lys Pro Gly Ala Ser Val Arg Leu Ser Cys Lys
        35                  40                  45
Ala Ser Gly Tyr Thr Phe Thr Glu Tyr Ile Ile His Trp Ile Lys Leu
    50                  55                  60
Arg Ser Gly Gln Gly Leu Glu Trp Ile Gly Trp Phe Tyr Pro Gly Ser
65                  70                  75                  80
Asn Asp Ile Gln Tyr Asn Ala Lys Phe Lys Gly Lys Ala Thr Leu Thr
                85                  90                  95
Ala Asp Lys Ser Ser Ser Thr Val Tyr Met Glu Leu Thr Gly Leu Thr
            100                 105                 110
Ser Glu Asp Ser Ala Val Tyr Phe Cys Ala Arg Arg Asp Asp Phe Ser
            115                 120                 125
Gly Tyr Asp Ala Leu Pro Tyr Trp Gly Gln Gly Thr Met Val Thr Val
        130                 135                 140
Ser Ser Gly Gly Gly Gly Ser Gly Gly Gly Gly Ser Gly Gly Gly Gly
145                 150                 155                 160
Ser Asp Ile Gln Met Thr Gln Ser Pro Ala Ser Leu Ser Val Ser Val
                165                 170                 175
Gly Glu Thr Val Thr Ile Thr Cys Arg Thr Asn Glu Asn Ile Tyr Ser
            180                 185                 190
Asn Leu Ala Trp Tyr Gln Gln Lys Gln Gly Lys Ser Pro Gln Leu Leu
        195                 200                 205
Ile Tyr Ala Ala Thr His Leu Val Glu Gly Val Pro Ser Arg Phe Ser
    210                 215                 220
Gly Ser Gly Ser Gly Thr Gln Tyr Ser Leu Lys Ile Thr Ser Leu Gln
225                 230                 235                 240
Ser Glu Asp Phe Gly Asn Tyr Tyr Cys Gln His Phe Trp Gly Thr Pro
                245                 250                 255
Cys Thr Phe Gly Gly Gly Thr Lys Leu Glu Ile Lys Arg Thr Val Ala
            260                 265                 270
Ala Pro Ser Glu Phe Asn Lys Ile Thr Pro Asn Leu Ala Glu Phe Ala
        275                 280                 285
Phe Ser Leu Tyr Arg Gln Leu Ala His Gln Ser Asn Ser Thr Asn Ile
    290                 295                 300
Phe Phe Ser Pro Val Ser Ile Ala Thr Ala Phe Ala Met Leu Ser Leu
305                 310                 315                 320
Gly Thr Lys Ala Asp Thr His Asp Glu Ile Leu Glu Gly Leu Asn Phe
            325                 330                 335
Asn Leu Thr Glu Ile Pro Glu Ala Gln Ile His Glu Gly Phe Gln Glu
            340                 345                 350
Leu Leu Arg Thr Leu Asn Gln Pro Asp Ser Gln Leu Gln Leu Thr Thr
        355                 360                 365
Gly Asn Gly Leu Phe Leu Ser Glu Gly Leu Lys Leu Val Asp Lys Phe
        370                 375                 380
Leu Glu Asp Val Lys Lys Leu Tyr His Ser Glu Ala Phe Thr Val Asn
385                 390                 395                 400
Phe Gly Asp Thr Glu Glu Ala Lys Lys Gln Ile Asn Asp Tyr Val Glu
                405                 410                 415
Lys Gly Thr Gln Gly Lys Ile Val Asp Leu Val Lys Glu Leu Asp Arg
            420                 425                 430
Asp Thr Val Phe Ala Leu Val Asn Tyr Ile Phe Phe Lys Gly Lys Trp
            435                 440                 445
Glu Arg Pro Phe Glu Val Lys Asp Thr Glu Glu Glu Asp Phe His Val
        450                 455                 460
Asp Gln Val Thr Thr Val Lys Val Pro Met Met Lys Arg Leu Gly Met
465                 470                 475                 480
Phe Asn Ile Gln His Cys Lys Lys Leu Ser Ser Trp Val Leu Leu Met
            485                 490                 495
```

```
Lys Tyr Leu Gly Asn Ala Thr Ala Ile Phe Phe Leu Pro Asp Glu Gly
            500                 505             510
Lys Leu Gln His Leu Glu Asn Glu Leu Thr His Asp Ile Ile Thr Lys
            515                 520             525
Phe Leu Glu Asn Glu Asp Arg Arg Ser Ala Ser Leu His Leu Pro Lys
        530                 535             540
Leu Ser Ile Thr Gly Thr Tyr Asp Leu Lys Ser Val Leu Gly Gln Leu
545                 550                 555             560
Gly Ile Thr Lys Val Phe Ser Asn Gly Ala Asp Leu Ser Gly Val Thr
                565                 570             575
Glu Glu Ala Pro Leu Lys Leu Ser Lys Ala Val His Lys Ala Val Leu
            580                 585             590
Thr Ile Asp Glu Lys Gly Thr Glu Ala Ala Gly Ala Met Phe Leu Glu
            595                 600             605
Ala Ile Pro Met Ser Ile Pro Pro Glu Val Lys Phe Asn Lys Pro Phe
        610                 615             620
Val Phe Leu Met Ile Glu Gln Asn Thr Lys Ser Pro Leu Phe Met Gly
625                 630                 635             640
Lys Val Val Asn Pro Thr Gln Lys Leu Glu Gly Glu Phe Glu Gln Lys
                645                 650             655
Leu Ile Ser Glu Glu Asp Leu Asn His His His His His
                660                 665
```

**Description**

L'invention est relative à des anticorps dirigés contre le récepteur lymphocytaire CD28 et à leurs fragments, et à leurs utilisations thérapeutiques, notamment dans le cadre de la régulation de l'activation des cellules T.

Une activation anormale des cellules T intervient dans la pathogenèse de nombreuses maladies autoimmunes, ainsi que dans les phénomènes de rejet de greffes où elle provoque le développement d'une réponse immune dirigée contre le greffon.

L'activation des lymphocytes T nécessite un signal activateur, induit par la reconnaissance par les récepteurs T (TCR) de l'antigène associé avec le complexe majeur d'histocompatibilité (CMH) de classe II et présenté par les cellules présentatrices de l'antigène (CPAg). Cette activation n'entraîne cependant la prolifération des cellules T et la sécrétion de cytokines immunomodulatrices spécifiques (telles que l'interleukine 2, l'interféron gamma ou l'interleukine 4), que si d'autres systèmes de co-stimulation T sont également activés.

L'un des systèmes les plus importants de régulation de l'activation des lymphocytes T est le système moléculaire B7/CD28/CTLA4. Ce système joue par exemple un rôle essentiel dans les mécanismes du rejet de greffe [WOODWARD et al., Transplantation, 66, 14-20, (1998)]. Les molécules B7.1 (CD80) et B7.2 (CD86) portées par les CPAgs peuvent activer le récepteur CD28 ainsi que le récepteur CTLA4 des lymphocytes T. L'activation du CD28 délivre au lymphocyte T un signal positif stimulant la cellule ; en revanche, l'activation du CTLA4 délivre un signal négatif conduisant à une non-réponse (anergie) [FALLARINO et al., J. Exp. Med., 188, 205-210, (1998)].

Les lymphocytes T au repos expriment une quantité importante de CD28, et très peu de CTLA4. Lors d'un premier contact cognitif entre une CPAg et un lymphocyte T, l'interaction CD28/B7 est privilégiée, ce qui active la cellule. Ce n'est que plusieurs heures après l'initiation de l'activation, du fait de l'augmentation de l'expression membranaire de CTLA4 dont l'affinité pour B7 est 5 à 10 fois supérieure à celle du CD28, que l'interaction B7/CD28 se déplace au profit d'une interaction B7/CTLA4.

Actuellement, pour bloquer l'activation des lymphocytes T, notamment dans le cadre des transplantations d'organes, on utilise principalement la cyclosporine. Malgré l'efficacité de ce médicament, la protection qu'il confère n'est cependant pas absolue. En outre, il agit en bloquant toutes les voies d'activation cellulaires dépendantes du calcium, et possède donc une activité biologique qui n'est pas strictement spécifique de lymphocytes T et entraîne un nombre important d'effets secondaires. Il est donc souhaitable de développer de nouveaux immunosuppresseurs au mode d'action défini et de spécificité plus grande.

Il a été postulé que l'inhibition sélective du signal agoniste délivré à la cellule T par le CD28 en laissant intact le système antagoniste constitué par le couple CTLA4/B7, par l'intermédiaire d'un blocage spécifique de l'interaction CD28/B7 permettrait de prévenir l'activation des lymphocytes T. Un tel blocage spécifique de l'interaction CD28/B7 peut être obtenu à l'aide d'un anticorps dirigé contre CD28.

Des anticorps anti-CD28 capables d'empêcher la liaison de CD28 avec B7 sont connus. Ils présentent toutefois l'inconvénient, lorsqu'ils sont utilisés sous leur forme native divalente, d'entraîner la dimérisation et l'activation de CD28 par leur liaison avec ce récepteur. Cependant, des fragments monovalents issus de ces anticorps sont capables de bloquer sans l'activer le récepteur CD28 [DAMLE et al., J. Immunol., 140, 1753-1761, (1988); NUNES et al., Int. Immunol., 5, 311-315, (1993) ; PAGES et al., J. Biol. Chem., 271, 9403, (1996)].

Il a ainsi été rapporté [PERRIN et al., J. Immunol. 163, 1704-1710, (1999)] que des fragments Fab issus d'un anticorps anti-CD28 pouvaient enrayer les symptômes cliniques de l'encéphalite expérimentale auto-immune induite chez la souris par l'administration de myéline ou le transfert de cellules T d'un animal atteint.

Des fragments monovalents, Fab ou scFv, dérivés d'un anticorps anti-CD28 sont potentiellement utilisables pour prévenir l'activation des lymphocytes T par l'intermédiaire d'un blocage spécifique de l'interaction CD28/B7.

Les fragments Fab résultent de l'action de la papaïne sur une molécule d'immunoglobuline, et contiennent chacun une chaîne légère et la première moitié d'une chaîne lourde ; les fragments scFv sont constitués des portions variables des chaînes lourdes et légères d'un anticorps, reliées entre elles par l'intermédiaire d'un lieur flexible [CLACKSON et al., Nature, 352, 624-628, (1991)], formant ainsi une protéine simple-chaîne.

Ces fragments monovalents présentent fréquemment une affinité pour l'antigène moins importante que celle des anticorps natifs, ce qui peut limiter leurs possibilités d'utilisation dans des applications diagnostiques ou thérapeutiques.

Les Inventeurs sont parvenus à sélectionner, parmi différents anticorps reconnaissant l'antigène CD28, un anticorps capable de bloquer l'interaction CD28/B7, et dont les fragments monovalents présentent une affinité pour l'antigène suffisante pour être utilisables, *in vitro* ou *in vivo*, pour bloquer le récepteur CD28 sans activation de ce récepteur.

Cet anticorps, dénommé CD28.3, est produit par l'hybridome déposé, selon les termes du traité de Budapest, le 28 novembre 2000 auprès de la CNCM (Collection Nationale de Cultures de Microorganismes, 25 rue du Docteur Roux, 75724 PARIS CEDEX 15) sous le numéro I-2582.

La présente invention a pour objet une protéine capable de se lier spécifiquement au récepteur lymphocytaire CD28 et de bloquer l'interaction CD28/B7, **caractérisé en ce qu'**elle comprend au moins les CDRs de la chaîne lourde et de la chaîne légère de l'immunoglobuline CD28.3, et qui est choisie parmi :

a) l'anticorps CD28.3 produit par l'hybridome CNCM I-2582 ;

b) les fragments Fv, Fab ou scFv de l'anticorps CD28.3 ;

c) les anticorps chimériques ou humanisés obtenus à partir des régions variables de CD28.3 ;

d) les fragments monovalents, Fv, Fab ou scFv des anticorps c) ci-dessus comprenant les CDRs de l'anticorps CD28.3 ,;

e) les protéines recombinantes comprenant un fragment b) ou d) et un polypeptide hétérologue.

Les CDRs (régions déterminant la complémentarité) sont les portions des régions variables d'une immunoglobuline impliquées dans la spécificité de reconnaissance de l'antigène.

Des protéines conformes à l'invention englobent ainsi notamment :

- des protéines associant au moins un fragment d'anticorps comprenant les CDRs de l'anticorps CD28.3, avec au moins un fragment d'anticorps comprenant les CDRs d'un anticorps de spécificité différente ; on citera à titre d'exemples, des immunoglobulines bi-spécifiques, des conjugués d'un fragment Fv ou Fab contenant les CDRs de CD28.3 avec un fragment Fv ou Fab d'un anticorps de spécificité différente, des « diabodies bi-spécifiques » résultant de l'association d'un fragment scFv contenant les CDRs de CD28.3 avec un fragment Fv ou Fab d'un anticorps de spécificité différente.

- des protéines associant au moins un fragment d'anticorps comprenant les CDRs de l'anticorps CD28.3, avec une molécule dotée d'activité pharmacologique (par exemple une toxine), ou de propriétés effectrices (par exemple un fragment Fc).

- des protéines associant au moins un fragment d'anticorps comprenant les CDRs de l'anticorps CD28.3, avec une molécule permettant de prolonger sa demi-vie plasmatique lors de son administration *in vivo* ; on peut par exemple associer ledit fragment d'anticorps avec un polypeptide hydrosoluble de masse moléculaire suffisante pour que la masse moléculaire du polypeptide de fusion ainsi obtenu soit supérieure au seuil de filtration rénale. Dans ce cas, on choisira un polypeptide qui contrairement aux fragments Fc, ne puisse pas s'associer en dimères, et qui ne possède pas d'activité effectrice propre susceptible d'entraîner des effets secondaires inopportuns. Des polypeptides possédant ces propriétés peuvent avantageusement être obtenus à partir de protéines sériques hydrosolubles, à savoir notamment l'albumine sérique, l'haptoglobuline, l'ITIH2 (inhibiteur inter-alpha (globuline), polypeptide H2), la transferrine, le CBG (protéine liant les corticostéroïdes), l'$\alpha$1 antitrypsine, l'ITIH4 (inhibiteur inter-alpha (globuline), polypeptide H4), l'AACT (alpha-1-antichymotrypsine), le TBG (globuline liant la thyroxine), le fibrinogène et la prothrombine, pour préparer des protéines de fusion avec des fragments scFv dérivés d'anticorps anti-CD28. On peut également conjuguer une protéine conforme à l'invention avec un polyol, par exemple le polyéthylène glycol, comme décrit par exemple dans le Brevet US 4,179,337.

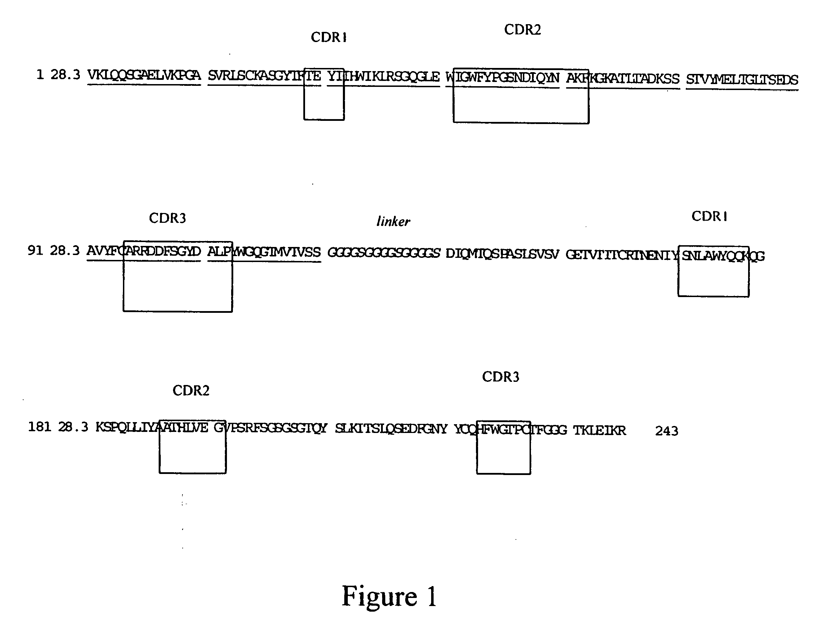

Un exemple d'une protéine conforme à l'invention est illustré par la Figure 1, qui représente un fragment scFv dérivé de l'anticorps CD28.3. Les séquences des CDRs de l'anticorps CD28.3 sont encadrées dans la séquence représentée sur la Figure 1.

La séquence nucléotidique codant pour ce fragment scFv est représentée dans la liste de séquences en annexe sous le numéro SEQ ID NO: 1 et la séquence peptidique correspondante est représentée sous le numéro SEQ ID NO: 2.

Des fragments Fv ou Fab conformes à l'invention peuvent être obtenus par les techniques classiques de digestion enzymatique, à partir de l'anticorps CD28.3.

Un plasmide contenant un polynucléotide codant pour un fragment scFv de CD 28.3, fusionné à un polynucléotide codant pour les acides aminés 53 à 425 de l'$\alpha$1 antitrypsine a été déposé, selon les termes du traité de Budapest, le 11 décembre 2001 auprès de la CNCM (Collection Nationale de Cultures de Microorganismes, 25 rue du Docteur Roux, 75724 PARIS CEDEX 15), sous le numéro I-2762.

Des protéines conformes à l'invention telles que des anticorps chimériques ou recombinants, des fragments scFv et leurs dérivés, etc. peuvent être obtenues, par les techniques classiques du génie génétique, telles que celles décrites par SAMBROOK et al., [MOLECULAR CLONING, A LABORATORY MANUAL, 2nd Ed., Cold Spring Harbor Laboratory Press, Cold Spring Harbor, N.Y., (1989)].

Des polynucléotides codant les régions variables de l'anticorps anti-CD28.3 peuvent par exemple être obtenus par clonage desdites régions variables à partir d'une banque d'ADNc de l'hybridome CD28.3, ou à partir du plasmide CNCM I-2762. Ils peuvent également être préparés totalement ou partiellement, par synthèse d'acides nucléiques, à partir des séquences nucléotidiques desdites régions variables. On peut par exemple synthétiser des polynucléotides codant les CDRs de CD28.3, et les incorporer dans les régions de charpente (FR pour « framework regions ») d'un autre anticorps, notamment d'un anticorps d'origine humaine, par les techniques, connues en elles-mêmes, de greffe de CDRs, telles que celles décrites par ROUTLEDGE et al., ["Reshaping antibodies for therapy", in PROTEIN ENGINEERING OF ANTIBODY MOLECULES FOR PROPHYLATIC AND THERAPEUTIC APPLICATIONS IN MAN, 13-44, Academic Titles, Nottingham, England (1993)] ou par ROGUSKA et al., Protein Engineering, 9(10), 895-904, (1996)].

La présente invention a également pour objet toute molécule d'acide nucléique codant une protéine conforme à l'invention comprenant les CDRs de l'anticorps CD28.3, ainsi que tout vecteur recombinant, notamment tout vecteur d'expression, comprenant ladite molécule d'acide nucléique.

La présente invention a également pour objet toute cellule exprimant une protéine conforme à l'invention comprenant les CDRs de l'anticorps CD28.3. Ceci englobe notamment l'hybridome CNCM I-2582, ainsi que les cellules-hôtes transformées par une molécule d'acide nucléique conforme à l'invention.

Des molécules d'acide nucléique conformes à l'invention peuvent avantageusement comprendre, outre une séquence codant une protéine conforme à l'invention, une séquence codant un peptide signal permettant la sécrétion de ladite protéine ; elles peuvent aussi comprendre une ou plusieurs séquence(s) codant un ou plusieurs peptide(s) marqueur(s) permettant la détection et/ou facilitant la purification de ladite protéine.

Des vecteurs d'expression conformes à l'invention comprennent au moins une séquence d'acide nucléique codant une protéine conforme à l'invention, associée à des éléments de contrôle de la transcription et de la traduction actifs dans la cellule-hôte choisie. Des vecteurs utilisables pour la construction de vecteurs d'expression conformes à l'invention sont connus en eux-mêmes, et seront choisis notamment en fonction de la cellule-hôte que l'on souhaite utiliser.

Des cellules-hôtes utilisables dans le cadre de la présente invention peuvent être des cellules procaryotes ou eucaryotes. Parmi les cellules eucaryotes utilisables, on citera en particulier des cellules végétales, des cellules de levure, telles que *Saccharomyces*, des cellules d'insecte, telles que les cellules de *Drosophila,* ou de *Spodoptera* et des cellules de mammifères telles que les cellules HeLa, CHO, 3T3, C127, BHK, COS, etc...

La construction de vecteurs d'expression conformes à l'invention, et la transformation des cellules-hôtes peut être effectuée par les techniques classiques de biologie moléculaire.

L'invention a également pour objet un procédé de production d'une protéine conforme à l'invention, **caractérisé en ce qu**'il comprend la mise en culture d'au moins une cellule conforme à l'invention, et la récupération de ladite protéine à partir de ladite culture.

Si la protéine est sécrétée, elle peut être récupérée directement à partir du milieu de culture ; sinon on procédera préalablement à la lyse des cellules.

La protéine peut ensuite être purifiée à partir du milieu de culture ou du lysat cellulaire, par des procédures classiques, connues en elles-mêmes de l'homme de l'art, par exemple par précipitation fractionnée, notamment précipitation au sulfate d'ammonium, électrophorèse, filtration sur gel, chromatographie d'affinité, etc.

Les protéines conformes à l'invention peuvent être utilisées *in vitro* pour étudier la réponse proliférative ou la différentiation de lymphocytes T répondant à une stimulation antigénique, virale, allogénique ou xénogénique. Elle peut être également utilisée *in vitro* pour induire la différenciation de lymphocytes T prélevés chez un patient, par exemple l'induction d'une tolérance vis-à-vis d'un antigène ou d'un alloantigène, destinés à être par la suite ré-administrés *in vivo*.

Elles peuvent également être utilisées pour l'obtention de médicaments, ou de réactifs de diagnostics.

Des protéines conformes à l'invention, monovalentes, c'est à dire possédant un seul site de liaison au récepteur CD28, sont utilisables dans tous les cas où l'on souhaite bloquer sélectivement ce récepteur sans l'activer, afin d'induire une immunosuppression.

Une protéine conforme à l'invention, comprenant un fragment monovalent dérivé d'un anticorps anti-CD28 peut notamment être utilisée pour l'obtention d'un médicament immunosuppresseur, bloquant sélectivement les phénomènes d'activation des cellules T impliquant le récepteur CD28, et ne présentant pas les inconvénients des immunosuppresseurs connus, tels que la cyclosporine.

L'immunosuppression T par blocage sélectif du CD28 par une protéine conforme à l'invention possède des applications dans toutes les pathologies dépendantes des lymphocytes T.

Il s'agit essentiellement du rejet de greffe, de la maladie du greffon contre l'hôte, des maladies auto-immunes à médiation lymphocytaire T, telles que le diabète de type I, ou la sclérose en plaques, et de l'hypersensibilité de type IV, qui intervient dans les phénomènes allergiques ainsi que dans la pathogenèse de maladies inflammatoires chroniques suivant une infection par un agent pathogène (notamment lèpre, tuberculose, leishmaniose, listérose, etc.).

La présente invention sera mieux comprise à l'aide du complément de description qui va suivre, qui se réfère à des exemples non-limitatifs de préparation et d'utilisation d'anticorps conformes à l'invention.

**EXEMPLE 1 : CHOIX D'UN ANTICORPS PRODUISANT DES FRAGMENTS MONOVALENTS PROPRIETES DE FRAGMENTS MONOVALENTS Fab ISSUS DE CD28.3.**

Certaines des propriétés de plusieurs anticorps anti-CD28 (CD28.1, CD28.2, CD28.3, CD28.4, CD28.5 et CD28.6) sont décrites dans la publication de NUNES et al. [Int. Immunol., 5, 311, (1993)]. Ces différents anticorps, qui ne sont pas accessibles au public, ont été fournis par le laboratoire de Daniel OLIVE (INSERM). Les propriétés de liaison à l'antigène des fragments monovalents Fab de ces différents anticorps ont été comparées.

5 mg de fragments Fab de chacun de ces anticorps ont été préparés par digestion à la papaïne (rapport molaire papaine/anticorps = 1/100) pendant 24 heures à 37°C, suivie d'inactivation de l'enzyme au iodoacétamide 0,03 M et de dialyse contre du PBS pour éliminer l'iodoacétamide.

**1) Liaison des fragments Fab à des cellules T Jurkat CD28+ :**

100 000 cellules Jurkat CD28+ en 100 μl sont incubées en PBS-BSA 1%-NaN3 0,1% à 4°C pendant 30 minutes avec des concentrations croissantes d'anticorps anti-CD28 ou de leurs fragments Fab. Après lavage, les cellules sont incubées de manière similaire avec un anticorps de chèvre anti-IgG de souris conjugué à la FITC, lavées, et analysées en cytofluorométrie.

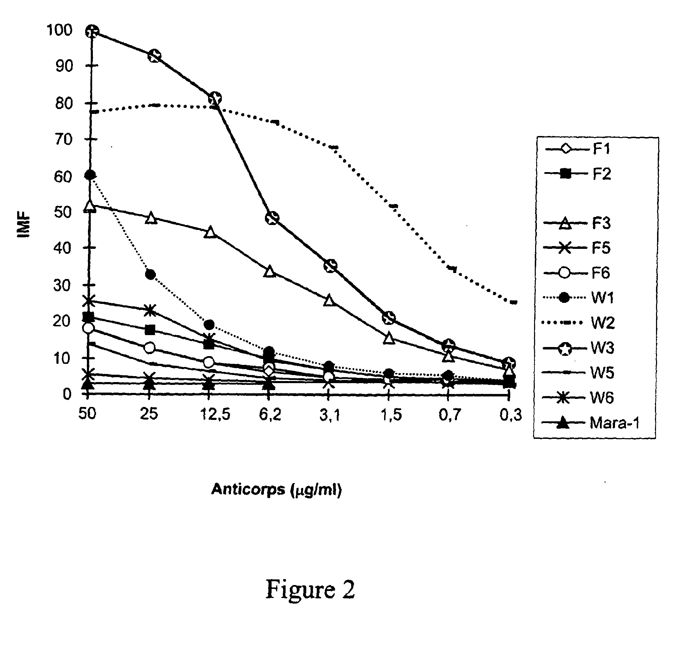

Les résultats sont illustrés par la Figure 2 :

Légende de la Figure 2 :

Axe des abscisses : concentration en anticorps ou fragments Fab
Axe des ordonnées : Intensité moyenne de fluorescence (IMF)

-◊- : F1 = Fragments Fab de l'anticorps CD28.1
-■- : F2 = Fragments Fab de l'anticorps CD28.2
-△- : F3= Fragments Fab de l'anticorps CD28.3
-✕- : F5 = Fragments Fab de l'anticorps CD28.5
-○- : F6 = Fragments Fab de l'anticorps CD28.6
..●.. : W1 = anticorps entier CD28.1
.._.. : W2 = anticorps entiers CD28.2
-✪- : W3 = anticorps entiers CD28.3
-_- : W5 = anticorps entiers CD28.5
-✶- : W6 = anticorps entiers CD28.6
-▲- : Mara-1 = contrôle négatif (IgG1 de souris).

Ces résultats montrent que parmi les fragments Fab, seuls ceux issus de CD28.3 sont capables de se lier de manière significative aux cellules Jurkat CD28+ à des concentrations inférieures à 10 μg/ml

**2) Effet des fragments Fab sur l'adhésion des cellules T Jurkat CD28+ à des cellules L murines transfectées exprimant la molécule B7-1. :**

4 x 10^5 cellules humaines T (Jurkatt, CD28-positives) marquées au $^{51}$Cr sont incubées pendant 2 heures dans une plaque de microtitration dans laquelle 10^5 cellules adhérentes LTK^- ou LB7^+ (fibroblastes murins transfectés avec B7.1 humain [PAGES et al., J. Biol. Chem., 271, 9403, (1996)] ont été ensemencées 24 heures auparavant. Ces incubations sont réalisées en présence des fragments Fab issus des anticorps CD28.1 à CD28.6, ou de l'anticorps CD28.3, dilués à différentes dilutions dans du tampon PBS sans $Ca^{2+}$ ni $Mg^{2+}$. Les cellules adhérentes après lavages sont quantifiées par lecture de la radioactivité résiduelle au compteur beta (PACKARD TOPCOUNT).

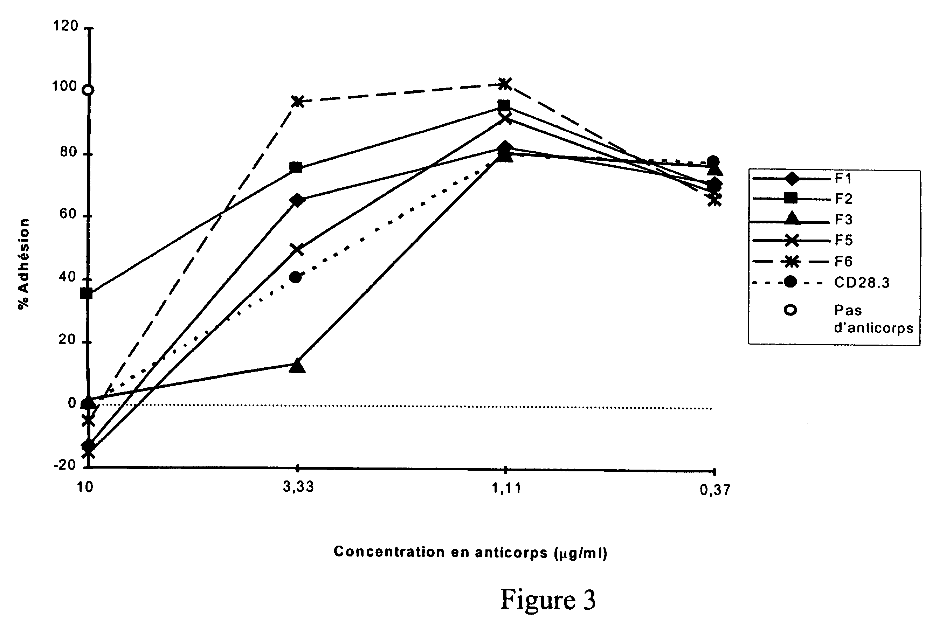

Les résultats sont illustrés par la Figure 3 :

Légende de la Figure 3 :

Axe des abscisses : pourcentage de cellules adhérentes
Axe des ordonnées : concentration en anticorps
-♦- : F1 = Fragments Fab de l'anticorps CD28.1
-■- : F2 = Fragments Fab de l'anticorps CD28.2
-▲- : F3 = Fragments Fab de l'anticorps CD28.3
-✕- : F5 = Fragments Fab de l'anticorps CD28.5
-*- : F6 = Fragments Fab de l'anticorps CD28.6
..●.. : Anticorps entier CD28.3
○ : Pas d'anticorps.

Ces résultats montrent que les fragments Fab issus de CD28.3 sont les plus efficaces pour inhiber les interactions CD28/B7. Ils inhibent l'adhésion à 90% à une concentration de 3 μg/ml, et avec une efficacité comparable à celle de l'anticorps CD28.3 entier, alors qu'à cette concentration, les fragments Fab issus des autres anticorps n'inhibent pas l'adhésion à plus de 50%.

**3) Effet des fragments Fab sur la prolifération en réaction lymphocytaire mixte :**

$10^5$ cellules mononucléées du sang périphérique sont mélangées avec $10^5$ cellules mononucléées allogéniques irradiées à 35 Gy, en présence de concentrations variables des anticorps CD28.1 à CD28.6 ou des fragments Fab issus de ces anticorps. La réponse proliférative dans ces cultures est évaluée après 3 jours, par incorporation de ($^3$H) thymidine pendant une durée de 16 heures.

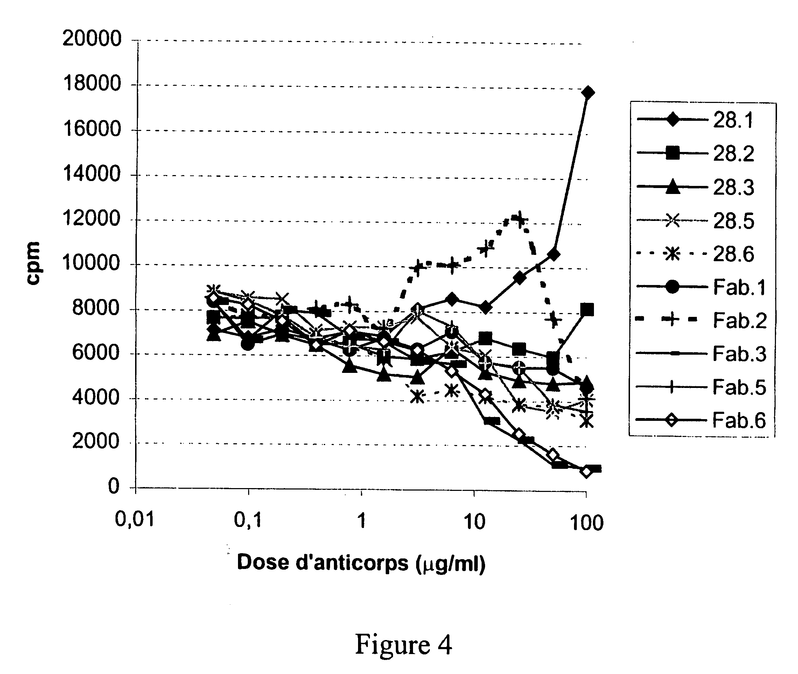

Les résultats sont illustrés par la Figure 4 :

Légende de la Figure 4 :

Axe des abscisse : concentration en anticorps
Axe des ordonnées : réponse proliférative (cpm)
Niveau basal de prolifération = 6500 cpm.

-♦- : 28.1 = anticorps CD28.1
-■- : 28.2 = anticorps CD28.2
-▲- : 28.3 = anticorps CD28.3
-✕- : 28.5 = anticorps CD28.5
..✳.. : 28.6 = anticorps CD28.6
-●- :Fab.1 = fragments Fab de l'anticorps CD28.1
..+.. : Fab.2 = fragments Fab de l'anticorps CD28.2
-_- : Fab.3 = fragments Fab de l'anticorps CD28.3
-+- : Fab.5 = fragment Fab de l'anticorps CD28.5
-◊- : Fab.6 = fragment Fab de l'anticorps CD28.6.

Ces résultats montrent que les fragments Fab issus de CD28.3 ou de CD28.6 sont les plus efficaces pour inhiber la prolifération des cellules mononucléées. Les anticorps entiers CD28.1 à CD28.6, testés en parallèle, n'ont aucun effet inhibiteur ou bien stimulent la prolifération de par leur action stimulatrice sur le CD28.

**Effet des fragments Fab issus de CD28.3 sur la prolifération induite par un super-antigène.**

Pour cette expérimentation des cellules T CD4+ répondeuses ont été mélangées avec des PBMC isogéniques irradiées, en présence de 50 ng/ml de toxine-1 du syndrome de choc toxique (TSST-1) qui stimule spécifiquement les cellules T Vβ2+, soit en l'absence d'anticorps, soit en présence d'anti-B7-1 (1 μg/ml), d'anti-B7-2 (0,5 μg/ml), de CTLA4Ig (10 μg/ml), ou de fragments Fab issus de CD28.3 (10 μg/ml).

La réponse proliférative dans ces cultures est évaluée après 1, 3, 6, et 8 jours, par incorporation de ($^3$H) thymidine pendant une durée de 16 heures.

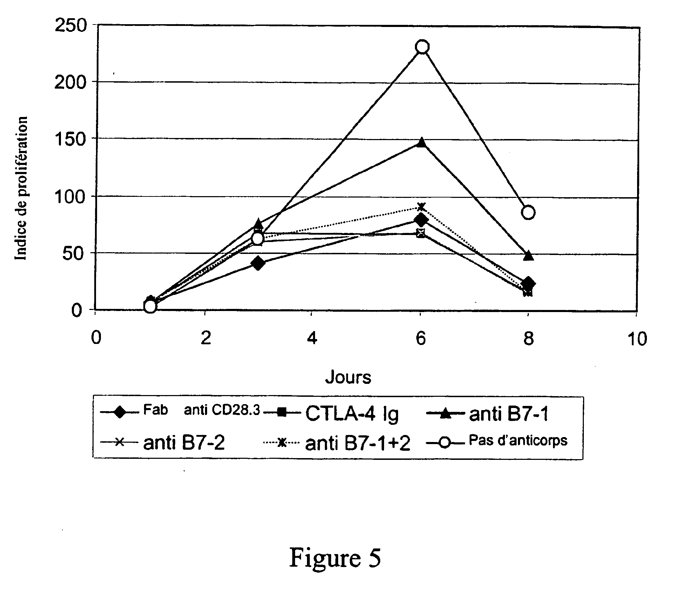

Les résultats sont illustrés par la Figure 5 :

Légende de la Figure 5 :

Axe des abscisses : temps de culture
Axe des ordonnées : indice de prolifération = IP

$$IP = \frac{\text{cpm réaction mixte lymphocytaire - cpm cellules stimulatrices irradiées seules}}{\text{cpm cellules répondeuses non stimulées}}$$

-♦- : Fab anti-CD28.3
-✕- : anti B7-2
-■- : CTLA-4 Ig
..*.. :anti-B7-1+2
-▲- : anti-B7-1

-○- : pas d'anticorps

TSST-1 induit une prolifération importante des cellules T CD4+. En présence d'anti-B7, de CTLA4Ig, ou des fragments Fab de CD28.3, on observe une inhibition de cette prolifération de 70% après 6 jours.

**Effet des fragments Fab issus de CD28.3 sur la production de cytokines.**

Afin de déterminer si les fragments Fab issus de CD28.3 pouvaient induire une déviation immune *in vitro*, une réaction lymphocytaire mixte (PBMC issues d'un donneur A/ PBMC irradiées issues d'un donneur B) a été effectuée, en présence de fragments Fab issus de CD28.3. $10^5$ cellules mononucléées du sang périphérique d'un donneur sont mélangées avec $10^5$ cellules mononucléées allogéniques irradiées à 35 Gy, et cultivées pendant 5 jours en présence ou en l'absence de 10 μg/ml de Fab issus de l'anticorps CD28.3.

L'ARN des cellules répondeuses a été extrait, et la quantité d'ARNm de cytokines a été évaluée par mesure quantitative du nombre de transcrits, rapportée à la quantité d'HPRT, à l'aide d'un TaqMan (Perkin Helmer).

On observe en présence de fragments Fab issus de CD28.3., une réduction de la production d'IFNy et d'IL2, et une augmentation de la production d'IL10. Cette déviation de la réponse immune suggère une orientation cers une réponse de type Th2. Ce résultat est inattendu dans la mesure où il a été rapporté que l'engagement de CTLA4 (qui est supposé intervenir lors du blocage de CD28 seul) conduit à une réponse de type Th1.

**Traitement *in vitro* de l'anticorps CD28.3 et des fragments Fab issus de celui-ci par les cellules T humaines.**

Une éventuelle internalisation des fragments Fab de l'anticorps CD28.3 dans les cellules T humaines a été recherchée, en comparaison avec l'anticorps entier CD28.3.

Des cellules T Jurkatt ont été incubées en milieu de culture avec 100 μg/ml d'anticorps CD28.3, à 37˚C ou à 0˚C. A différents temps, les cellules ont été lavées avec du tampon PBS froid contenant 0,1% de sérum-albumine bovine, et du NaN3, afin de bloquer la motilité membranaire. Les anticorps liés ont été révélés avec un anticorps secondaire de chèvre anti-souris, marqué à la fluorescéine. Les cellules ont été montées dans du MOVIOL et analysées par microscopie confocale.

On observe ainsi que les anticorps CD28.3 entiers se liant aux cellules T Jurkatt sont capturés et disparaissent de la surface cellulaire à 37˚C, mais pas à 0˚C. Au contraire, les fragments Fab restent fixés en surface de la cellule. Ceci indique que la fixation des anticorps divalents CD28.3 entraîne la dimérisation de CD28, ce qui conduit à leur entrée dans la cellule, alors que les fragments monovalents Fab, qui n'induisent pas cette dimérisation, demeurent en surface.

**EXEMPLE 2 : PROPRIETES D'UN FRAGMENT scFv DERIVE DE L'ANTICORPS CD28.3.**

La Figure 1 représente la séquence nucléotidique et la séquence polypeptidique déduite d'un fragment scFv dérivé de l'anticorps CD28.3. Les portions de cette séquence correspondant au fragment variable de la chaîne lourde et de la chaîne légère sont représentés en lettres capitales. La séquence correspondant au fragment variable de la chaîne légère est en outre souligné. La séquence du lieur est représentée en lettres minuscules. Les séquences des CDRs de la chaîne lourde et de la chaîne légère sont encadrées.

La séquence nucléotidique codant ce fragment scFv est également représentée dans la liste de séquences en annexe, sous le numéro SEQ ID NO:˚1.

L'ADNc codant ce fragment scFv a été inséré dans le vecteur pIG6 (Biochemisches Institut, Universität Zurich). Ce vecteur comprend notamment un marqueur de résistance à l'ampicilline, et une cassette d'expression qui comprend un promoteur lac inductible, sous contrôle duquel sont placés : une séquence codant un peptide signal ompA, une séquence codant un peptide marqueur de séquence (code 1 lettre) DYKD, une séquence codant un peptide marqueur c-myc, et une séquence codant un marqueur polyhistidine-5.

L'ADNc codant le fragment scFv décrit ci-dessus a été introduit entre les sites EcoRI et EcoRV de pIG6, en aval de la séquence codant le peptide DYKD et en amont de la séquence codant le marqueur c-myc.

La construction obtenue est dénommée pIg6-28.3.

**Production en cellules procaryotes**

Le vecteur pIg6-28.3 a été utilisé pour transformer des cellules *E.coli* JM83. Les cellules sont cultivées à 25˚C, jusqu'à une DO$_{550}$ de 0,5. Après induction par l'IPTG, le fragment scFv est produit sous forme soluble dans le périplasme. Il apparaît après électrophorèse et transfert de Western, sous forme d'une bande à environ 30 kDa.

Il est purifié à partir des extraits périplasmiques des bactéries, obtenus après choc osmotique en 50 mM Tris-

CI, et ultracentrifugation du matériel insoluble, par chromarographie sur matrice de NI-NTA et échange d'ions sur DEAE-sépharose.

La liaison des fragments scFv présents dans l'éluat de la colonne de NiNTA à des cellules Jurkat CD28+ est comparable à celle obtenue avec des fragments Fab obtenus à partir de l'anticorps CD28.3 par digestion à la papaïne.

### Production en cellules eucaryotes

Le vecteur pSec-28.3 a été utilisé pour transfecter des cellules Cos. Les cellules sont cultivées à 37˚C pendant 3 jours. Le fragment scFv est produit sous forme soluble dans le surnageant. Ce surnageant inhibe la réaction mixte lymphocytaire :$10^5$ cellules mononucléées du sang périphérique d'un donneur sain sont mélangées avec $10^5$ cellules mononucléées du sang périphérique d'un autre donneur sain allogénique. La réponse proliférative dans ces cultures est évaluée après 5 jours par incorporation de ($^3$H)Thymidine pendant une durée de 16 heures. On observe une inhibition importante de l'incorporation dépendant de la dilution du surnageant utilisée. Un surnageant contrôle ne présente pas d'activité inhibitrice de la prolifération.

### EXEMPLE 3 : OBTENTION D'UNE PROTEINE DE FUSION COMPRENANT UN FRAGMENT scFv DE CD28.3.

La séquence nucléotidique codant le fragment scFv décrit à l'Exemple 2 a été liée à l'extrémité 5' d'une portion de l'ADNc de l'α1-antitrypsine humaine (numéro d'accès GENBANK K01396) correspondant aux acides aminés 53 à 425, par l'intermédiaire d'un peptide charnière, de séquence VAAPS. La séquence résultante est représentée dans la liste de séquences en annexe sous le numéro SEQ ID NO: 3, et le polypeptide correspondant sous le numéro SEQ ID NO: 4.

### EXEMPLE 4 : CONSTRUCTION DE VECTEURS D'EXPRESSION COMPRENANT LA SEQUENCE CODANT POUR L'α-1 ANTITRYPSINE ET PERMETTANT L'INTRODUCTION D'UNE SEQUENCE CODANT UN FRAGMENT scFv

#### Vecteur d'expression procaryote :

Le vecteur pIG6 a été utilisé (Biochemisches Institut, Universität Zurich). Ce vecteur comprend notamment un marqueur de résistance à luote ampicilline, et une cassette d'expression qui comprend un promoteur lac inductible, sous contrôle duquel sont placés : une séquence codant un peptide signal ompA, une séquence codant un peptide marqueur de séquence (code 1 lettre) DYKD, une séquence codant un peptide marqueur c-myc, et une séquence codant un marqueur polyhistidine-5.

L'ADNc codant un fragment d'α1-antitrypsine humaine correspondant aux acides aminés 53 à 425, a été introduit entre les sites EcoRI et EcoRV de pIG6, en aval de la séquence codant le peptide DYKD et en amont de la séquence codant le marqueur c-myc.

La Figure 1 schématise la construction obtenue, dénommée pIg6-Haat.

#### Vecteur d'expression eucaryote :

Le vecteur pSECTagB (Invitrogen, De Schelp, Pays Bas) a été utilisé. Ce vecteur comprend notamment un marqueur de résistance à l'ampicilline, un marqueur de résistance à la zéocine, et une cassette d'expression qui comprend un promoteur CMV, sous contrôle duquel sont placés : une

séquence codant un peptide signal de la chaîne légère IgG Kappa, une séquence codant un peptide marqueur c-myc, et une séquence codant un marqueur polyhistidine-6.

L'ADNc codant un fragment d'α1-antitrypsine humaine correspondant aux acides aminés 53 à 425, a été introduit entre les sites BamHI et EcoRI du vecteur PSEC B Tag, en amont de la séquence codant le marqueur c-myc.

La Figure 2 schématise la construction obtenue, dénommée pSecHaat.

### EXEMPLE 5 : CONSTRUCTION DE VECTEURS D'EXPRESSION INTEGRANT LA SEQUENCE CODANT LA PROTEINE DE FUSION scFv CD28.3/α1-ANTITRYPSINE

#### Vecteur d'expression procaryote :

L'ADNc codant la protéine de fusion ScFv CD28.3/α1-antitrypsine décrite à l'exemple 3 ci-dessus a été introduit entre les sites EcoRI et XhoI de pIG6, en aval de la séquence codant le peptide DYKD et en amont de la séquence codant le marqueur c-myc.

La Figure 3 schématise la construction obtenue, dénommée pIg6-28.3Haat.

**Vecteur d'expression eucaryote :**

L'ADNc codant la protéine de fusion ScFv CD28.3/α1-antitrypsine décrite à l'exemple 3 ci-dessus a été introduit entre les sites BamHI et XhoI du vecteur PSEC B Tag, en amont de la séquence codant le marqueur c-myc.

La Figure 4 schématise la construction obtenue, dénommée pSec-28.3Haat.

Ce vecteur, hébergé dans *E.coli* DH5α, a été déposé le 11 décembre 2001 auprès de la CNCM, sous le numéro I-2762.

**EXEMPLE 6 : EXPRESSION ET PURIFICATION DES PROTEINES DE FUSION**

**En cellules procaryotes :**

Le vecteur pIg6-28.3Haat a été utilisé pour transformer des cellules *E.coli* JM83. Les cellules sont cultivées à 25˚C, jusqu'à une $DO_{550}$ de 0,5. Après induction par l'IPTG, la protéine est produite sous forme soluble dans le périplasme. Elle apparaît après électrophorèse et transfert de Western, sous forme d'une bande à environ 74 kDa.

Elle peut être purifiée à partir des extraits périplasmiques en utilisant une matrice de chromatographie d'affinité NI-NTA, et/ou une matrice de chromatographie d'affinité anti-c-myc. Elle peut également être purifiée à partir d'une colonne d'affinité anti-α1-antitrypsine.

**En cellules eucaryotes :**

Le vecteur pSec-28.3Haat a été utilisé pour transfecter des cellules CHO par lipofection. Les cellules sont cultivées en présence de 200 μg/ml de zéocine en milieu MEM contenant 10% de sérum de veau foetal.

La protéine est sécrétée dans le milieu de culture.

Après séparation par électrophorèse, transfert de Western, et révélation par un anticorps anti-c-myc elle apparaît sous forme d'une bande à environ 80 kDa.

**EXEMPLE 7 : TESTS D'ACTIVITE D'UNE PROTEINE DE FUSION scFv /α1-ANTITRYPSINE**

L'activité anti-CD28 de la protéine de fusion scFv CD28.3/α1-antitrypsine obtenue à l'exemple 6 ci-dessus est évaluée par sa liaison à la molécule CD28, ou à des cellules exprimant CD28 sur leur membrane, et son absence de liaison à des cellules qui n'expriment pas CD28.

L'activité immunosuppressive de la protéine de fusion scFv CD28.3/α1-antitrypsine obtenue à l'exemple 6 ci-dessus est évaluée par l'inhibition de l'adhésion à B7, et l'inhibition de l'activation induite du lymphocyte T.

Ces activités anti-CD28 et immunosuppressive ont été mesurées par les tests suivants :

**Activité anti CD28**

Mesure au biosenseur des paramètres de liaison à CD28:

Du CD28 humain recombinant a été immobilisé sur le détecteur du biosenseur (BIACORE). Une protéine de fusion scFv CD28.3/α1-antitrypsine obtenue comme décrit à l'exemple 6 ci-dessus a été mise en contact avec le détecteur. Les paramètres de liaison sont : KA (1/M)$2.86^e9$ ; KD (M) : $3.49^e$-10. En comparaison, ces paramètres mesurés pour le fragment Fab de l'anticorps CD28.3 sont : KA (1/M) : $9.69^e8$ ; KD (M) : $1.03^e$-9. L'affinité pour CD28 du fragment Fab de l'anticorps CD28.3 et de la protéine de fusion sont donc comparables.

Test de reconnaissance spécifique de CD28 en cytofluorométrie :

$10^5$ cellules Jurkat (CD28+) et U937 (CD28-) sont incubées en PBS-BSA 1%-NaN3 0,1% à 4˚C pendant 1 heure avec des concentrations croissantes de la protéine de fusion scFv CD28.3/α1-antitrypsine. Après lavage, les cellules sont incubées avec un anticorps de lapin anti-alpha-1-antitrypsine, puis avec un anticorps de chèvre anti-lapin conjugué à la FITC, lavées, et analysées en cytofluorométrie. On observe une liaison dépendante de la dose sur les cellules Jurkat (CD28+) et pas de liaison sur les cellules U937 (CD28-). Ceci montre la spécificité de la protéine de fusion pour la molécule CD28 et son absence de réactivité envers d'autres molécules exprimées par des cellules hématopoïétiques humaines.

**Activité immunosuppressive**

Test d'adhésion CD28/B7-dépendant :

4 x $10^5$ cellules humaines T (Jurkatt, CD28-positives) marquées au $^{51}$Cr sont incubées pendant 2 heures dans une plaque de microtitration dans laquelle $10^5$ cellules adhérentes LTK$^-$ ou LB7$^+$ (fibroblastes murins transfectés avec B7.1 humain [PAGES et al., J. Biol. Chem., 271, 9403 (1996)] ont été ensemencées 24 heures auparavant. Ces incubations sont réalisées en absence ou en présence de la protéine de fusion scFv CD28.3/$\alpha$1-antitrypsine, diluée à différentes concentrations dans du tampon PBS sans Ca$^{2+}$ ni Mg$^{2+}$. Les cellules adhérentes après lavages sont quantifiées par lecture de la radioactivité résiduelle au compteur beta (PACKARD TOPCOUNT). On observe une inhibition de l'adhésion en présence de la protéine de fusion scFv CD28.3/$\alpha$1-antitrypsine, inhibition directement dépendante de la dose de protéine de fusion utilisée.

Inhibition de l'activation :

5 x $10^4$ cellules T (polyclonales humaines, déplétées en cellules CD11b) sont stimulées avec 1 x $10^4$ cellules d'hydridome OKT3 (anti-CD3) irradiées, ou avec des cellules B CD28$^-$ allogéniques (déplétées en cellules CD28$^+$) en absence ou en présence de quantités variables de la protéine de fusion scFv CD28.3/$\alpha$1-antitrypsine. La réponse proliférative dans ces cultures est évaluée après 3 jours lorsque la stimulation est effectuée par des anti-CD3, ou après 7 jours lorsque la stimulation est effectuée par des cellules allogéniques, par incorporation de ($^3$H)Thymidine pendant une durée de 16 heures. On observe une inhibition importante de l'incorporation en présence de la protéine de fusion scFv CD28.3/$\alpha$1-antitrypsine, inhibition directement dépendante de la dose de protéine de fusion utilisée.

Inhibition de la réaction mixte lymphocytaire :

$10^5$ cellules mononucléées du sang périphérique d'un donneur sain sont mélangées avec $10^5$ cellules mononucléées du sang périphérique de un autre donneur sain allogénique. La réponse proliférative dans ces cultures est évaluée après 5 jours par incorporation de ($^3$H) Thymidine pendant une durée de 16 heures. On observe une inhibition importante de l'incorporation en présence de la protéine de fusion scFv CD28.3/$\alpha$1-antitrypsine, inhibition directement dépendante de la dose de protéine de fusion utilisée.

**Revendications**

1. Protéine capable de se lier spécifiquement au récepteur lymphocytaire CD28 et de bloquer l'interaction CD28/B7, **caractérisée en ce qu'**elle comprend au moins les CDRs de la chaîne lourde et de la chaîne légère de l'immunoglobuline CD28.3, produite par l'hybridome CNCM I-2582, et **en ce qu'**elle est choisie parmi :

   a) l'anticorps CD28.3 produit par l'hybridome CNCM I-2582 ;
   b) les fragments Fv, Fab, ou scFv de l'anticorps CD28.3 ;
   c) les anticorps chimériques ou humanisés obtenus à partir des régions variables de CD28.3 ;
   d) les fragments Fv, Fab, ou scFv d'un anticorps chimérique ou humanisé c);
   d) les protéines recombinantes comprenant un fragment b) ou d) et un polypeptide hétérologue.

2. Protéine selon la revendication 1, **caractérisée en ce qu'**il s'agit d'une protéine recombinante comprenant :

   - un fragment scFv de l'anticorps CD28.3 et
   - un polypeptide hétérologue obtenu à partir de l'$\alpha$1-antitrypsine.

3. Molécule d'acide nucléique codant une protéine selon une quelconque des revendications 1 ou 2.

4. Vecteur d'expression, comprenant une molécule d'acide nucléique selon la revendication 3.

5. Vecteur d'expression selon la revendication 4, **caractérisé en ce qu'**il s'agit du plasmide CNCM I-2762.

6. Cellule exprimant une protéine selon une quelconque des revendications 1 ou 2.

7. Cellule selon la revendication 6, **caractérisée en ce qu'**il s'agit de l'hybridome CNCM I-2582.

8. Cellule selon la revendication 6, **caractérisée en ce qu'**il s'agit d'une cellule transformée par une molécule d'acide nucléique selon la revendication 3.

9. Procédé de préparation d'une protéine selon une quelconque des revendications 1 ou 2, **caractérisé en ce qu'**il comprend la mise en culture d'au moins une cellule selon une quelconque des revendications 6 à 8, et la récupération de ladite protéine à partir de ladite culture.

10. Utilisation d'une protéine selon une quelconque des revendications 1 ou 2, pour l'obtention d'un médicament.

11. Utilisation selon la revendication 10, **caractérisée en ce que** ladite protéine possède un seul site de liaison au récepteur CD28, et **en ce que** ledit médicament est un immunosuppresseur, bloquant sélectivement l'activation des cellules T par l'intermédiaire du récepteur CD-28

12. Utilisation selon la revendication 11, **caractérisée en ce que** ledit médicament est destiné au traitement d'une pathologie choisie parmi le rejet de greffe, la maladie du greffon contre l'hôte, les maladies auto-immunes à médiation lymphocytaire T, les phénomènes allergiques, les maladies inflammatoires chroniques.

**Claims**

1. Protein capable of binding specifically to the lymphocyte CD28 receptor and of blocking CD28/B7 interaction, **characterised in that** it comprises at least the CDRs of the heavy chain and of the light chain of immunoglobulin CD28.3, produced by the hybridoma CNCM I-2582, and **in that** it is selected from:

   a) the antibody CD28.3 produced by the hybridoma CNCM I-2582;
   b) Fv, Fab or scFv fragments of the antibody CD28.3;
   c) chimeric or humanised antibodies obtained from variable regions of CD28.3;
   d) Fv, Fab or scFv fragments of a chimeric or humanised antibody c);
   e) recombinant proteins comprising a fragment b) or d) and a heterologous polypeptide.

2. Protein according to claim 1, **characterised in that** it is a recombinant protein comprising:

   - an scFv fragment of the antibody CD28.3 and
   - a heterologous polypeptide obtained from $\alpha$1-antitrypsin.

3. Nucleic acid molecule coding for a protein according to either claim 1 or claim 2.

4. Expression vector comprising a nucleic acid molecule according to claim 3.

5. Expression vector according to claim 4, **characterised in that** it is the plasmid CNCM I-2762.

6. Cell expressing a protein according to either claim 1 or claim 2.

7. Cell according to claim 6, **characterised in that** it is the hybridoma CNCM I-2582.

8. Cell according to claim 6, **characterised in that** it is a cell transformed by a nucleic acid molecule according to claim 3.

9. Process for the preparation of a protein according to either claim 1 or claim 2, **characterised in that** it comprises culturing at least one cell according to any one of claims 6 to 8 and recovering said protein from said culture.

10. Use of a protein according to either claim 1 or claim 2 to obtain a medicament.

11. Use according to claim 10, **characterised in that** said protein has a single CD28 receptor binding site, and **in that** said medicament is an immunosuppressant which selectively blocks the T cell activation by way of the CD28 receptor.

12. Use according to claim 11, **characterised in that** said medicament is intended for the treatment of a pathology selected from graft rejection, graft-versus-host disease, T lymphocyte-mediated autoimmune diseases, allergic phenomena, and chronic inflammatory diseases.

**Patentansprüche**

1. Protein, das geeignet ist sich spezifisch an den lymphozytären CD28-Rezeptor zu binden und die Interaktion CD28/B7 zu blockieren, **dadurch gekennzeichnet, dass** es wenigstens die CDR der schweren Kette umfasst und die der leichten Kette des Immunglobulins CD28.3, das es durch das Hybridom CNCM 1-2582 hergestellt wird, und **dadurch**, dass es ausgewählt ist aus:

   a) dem durch das Hybridom CNCM 1-2582 hergestellten CD28.3-Antikörper;
   b) den Fragmenten Fv, Fab oder scFv des CD28.3-Antikörpers;
   c) den chimären oder humanen Antikörpern, der ausgehend von den variablen Regionen des CD28.3 erhalten werden;
   d) den Fragmenten Fv, Fab oder scFv eines chimären oder humanen Antikörpers c);
   e) den ein Fragment b) oder d) und ein heterologes Polypeptid umfassenden rekombinanten Proteinen.

2. Protein nach Anspruch 1, **dadurch gekennzeichnet, dass** es sich um ein rekombinantes Protein handelt, das folgendes umfasst:

   - ein scFv-Fragment des CD28.3-Antikörpers und
   - ein heterologes Polypeptid, das ausgehend von l'$\alpha$1-Antitrypsin erhalten wird.

3. Nukleinsäuremolekül, das ein Protein nach einem der Ansprüche 1 oder 2 codiert.

4. Expressionsvektor, umfassend ein Nukleinsäuremolekül nach Anspruch 3.

5. Expressionsvektor nach Anspruch 4, **dadurch gekennzeichnet, dass** es ich um das Plasmid CNCM 1-2762 handelt.

6. Zelle, die ein Protein nach einem der Ansprüche 1 oder 2 exprimiert.

7. Zelle nach Anspruch 6, **dadurch gekennzeichnet, dass** es sich um das Hybridom CNCM 1-2582 handelt.

8. Zelle nach Anspruch 6, **dadurch gekennzeichnet, dass** es sich um eine durch ein Nukleinsäuremolekül nach Anspruch 3 transformierte Zelle handelt.

9. Herstellungsverfahren für ein Protein nach einem der Ansprüche 1 oder 2, **dadurch gekennzeichnet, dass** es das Kultivieren wenigstens einer Zelle nach einem der Ansprüche 6 bis 8 umfasst und die Gewinnung des genannten Proteins ausgehend von der Kultur.

10. Verwendung eines Proteins nach einem der Ansprüche 1 oder 2 zum Erhalt eines Arzneimittels.

11. Verwendung nach Anspruch 10, **dadurch gekennzeichnet, dass** das Protein eine einzelne Bindungsstelle an dem CD28-Rezeptor besitzt und das Arzneimittel ein Immunsuppressivum ist, das selektiv die Aktivierung der T-Zellen mittels des CD28-Rezeptors blockiert.

12. Verwendung nach Anspruch 11, **dadurch gekennzeichnet, dass** das Arzneimittel für die Behandlung eines pathologischen Leidens bestimmt ist, ausgewählt aus Transplantatabstoßung, Transplantat versus Wirt-Krankheit, Autoimmunkrankheiten mit Vermittlung von T-Lymphocyten, allergischen Phänomene, chronisch inflammatorischen Krankheiten.

CDR1      CDR2

1 28.3 VKLQQSGAELVKPGA SVRLSCKASGYIFTE YILHWIKLRSGQGLE WIGWFYPGSNDIQYN AKFKGKATLTADKSS STVYMELTGLTSEDS

CDR3      *linker*      CDR1

91 28.3 AVYFCARRDDFSGYD ALPYWGQGIMVIVSS *GGGGSGGGGSGGGGS* DIQMTQSPASLSVSV GETVTITCRINENIY SNLAWYQKQG

CDR2      CDR3

181 28.3 KSPQLLIYAATHLVE GVPSRFSGSGSGTQY SLKITSLQSEDFGNY YCQHFWGTPCTFGGG TKLEIKR    243

## Figure 1

Figure 2

Figure 3

EP 1 345 969 B1

Figure 4

EP 1 345 969 B1

Figure 5

# EP 1 345 969 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 4179337 A

### Littérature non-brevet citée dans la description

- **WOODWARD et al.** *Transplantation,* 1998, vol. 66, 14-20
- **FALLARINO et al.** *J. Exp. Med.,* 1998, vol. 188, 205-210
- **DAMLE et al.** *J. Immunol.,* 1988, vol. 140, 1753-1761
- **NUNES et al.** *Int. Immunol.,* 1993, vol. 5, 311-315
- **PAGES et al.** *J. Biol. Chem.,* 1996, vol. 271, 9403
- **PERRIN et al.** *J. Immunol.,* 1999, vol. 163, 1704-1710
- **CLACKSON et al.** *Nature,* 1991, vol. 352, 624-628
- **SAMBROOK et al.** MOLECULAR CLONING, A LABORATORY MANUAL. Cold Spring Harbor Laboratory Press, 1989
- Reshaping antibodies for therapy. **ROUTLEDGE et al.** PROTEIN ENGINEERING OF ANTIBODY MOLECULES FOR PROPHYLATIC AND THERAPEUTIC APPLICATIONS IN MAN. Academic Titles, 1993, vol. 13-44
- **ROGUSKA et al.** *Protein Engineering,* 1996, vol. 9 (10), 895-904
- **NUNES et al.** *Int. Immunol.,* 1993, vol. 5, 311